# EUROPEAN PATENT SPECIFICATION

(11) **EP 4 133 989 B1**
(45) Date of publication and mention of the grant of the patent: **10.12.2025**
(21) Application number: 21788562.3
(22) Date of filing: 16.03.2021
(51) Int. Cl.: A61B 1/005

(54) **ELECTRO-DEFORMATION BENDING ENDOSCOPE**
BIEGEENDOSKOP MIT ELEKTROVERFORMUNG
ENDOSCOPE FLEXIBLE PAR ÉLECTRODÉFORMATION

(30) Priority: 13.04.2020 CN 202010284028
(43) Date of publication of application: 15.02.2023
(73) Proprietor: Zhuhai Vision Medical Technology Co., Ltd, Zhuhai, Guangdong 519031 (CN)
(72) Inventor: LI, Tianbao, Zhuhai, Guangdong 519031 (CN); YANG, Kai, Zhuhai, Guangdong 519031 (CN); CHEN, Shu, Zhuhai, Guangdong 519031 (CN); LIU, Fayang, Zhuhai, Guangdong 519031 (CN)
(74) Representative: Araujo, Daniel
(86) International application number: PCT/CN2021/081040
(87) International publication number: WO 2021/208657

(56) References cited:
- WO-A1-2017/136729
- CN-A- 1 870 930
- CN-A- 101 010 027
- CN-A- 101 179 980
- CN-A- 101 198 370
- CN-A- 101 776 796
- CN-A- 101 995 653
- CN-A- 104 013 377
- CN-A- 105 188 502
- CN-A- 108 882 838
- CN-A- 111 528 768
- JP-A- H05 184 531
- US-A1- 2009 048 488

## Description

### TECHNICAL FIELD

The present invention relates to the field of endoscopes, and more particularly, to an electro-deformation bending endoscope.

### BACKGROUND

Endoscope is an important instrument which may be sent into a cavity of a human body for visual examination and surgical treatment. The endoscope is applied to a plurality of fields such as examination of gastrointestinal diseases, examination of pancreas and biliary tract diseases, examination of urinary tract and the like. The endoscope is classified clinically according to whether a direction of an endoscope body can be changed or not, and is divided into hard endoscope and soft endoscope. The hard endoscope is a prism optical system, has the greatest advantage of clear imaging, may be matched with a plurality of working channels and select a plurality of perspectives, is convenient to operate, but has blind spots of vision since its direction cannot be adjusted. Chinese patent application CN200310115365.0 discloses a ureteroscope, wherein a tail end of a body of the ureteroscope is designed as two sections, and the two sections are connected by a joint controllable to rotate, so that the tail end of the body of the ureteroscope can be bent by 180 degrees in two opposite directions. By adopting the design of dividing the tail end of the body of the ureteroscope into the two sections and connecting the two sections by the joint controllable to rotate, the tail end of the body of the ureteroscope can be bent by 180 degrees in two opposite directions respectively. A mechanical transmission mode is adopted to control the tail end of the body of the ureteroscope to rotate, so the endoscope has a complex structure, a mass and a size of the endoscope body are increased, and the endoscope is not facilitated to operate.

Patent document US 2009/048488 A1 discloses comprising a bending portion in the vicinity of an insertion portion is formed with an EPAM actuator. When bending instruction in an arbitrary direction is performed by a bending amount instruction means, a bending amount control means bends the EPAM actuator by driving EPAM drive means and applying a driving voltage between electrodes in response to the bending instruction.

Patent document JP H05 184531 A1 discloses a medical tube having: a storage chamber filled with an electrolyte solution formed in the tube; a pair of energizing electrodes arranged in a main body of the medical tube; an actuating member made of a mechanochemical substance that bends when a voltage is applied through the electrolyte solution; and an energizing control unit that applies a voltage to the energizing electrodes is provided.

### SUMMARY

The present invention which is defined in the appended claims aims at least solving one of the technical problems in the existing technology, and providing an electro-deformation bending endoscope, which has a simple structure and can realize a function of adjusting a bending angle of the endoscope without increasing a mass and a size of the endoscope.

The present invention provides an electro-deformation bending endoscope, including a handle portion and an insertion portion provided below the handle portion, wherein the insertion portion includes a straight tube and an electro-deformation bending tube, a lower end of the straight tube is coaxially fixed with an upper end of the electro-deformation bending tube, the electro-deformation bending tube includes a plurality of deformation bending units connected end to end, each deformation bending unit includes at least two electro-deformation pieces made of an electro-deformation material, an insulating layer is provided between the electro-deformation pieces, electrodes are provided at two ends of each electro-deformation piece, and the electrodes are connected with a voltage generating unit through a wire.

Beneficial effects: the endoscope of the present invention includes the handle portion and the insertion portion provided below the handle portion, the insertion portion includes the straight tube and the electro-deformation bending tube, the electro-deformation bending tube includes the at least two electro-deformation pieces, the electro-deformation pieces are made of the electro-deformation material and deform once an electroactive polymer is subjected to electrical stimulation; the electrodes are provided at the two ends of each electro-deformation piece, and voltage is applied to the electrodes to control the electro-deformation pieces to bend, thereby bending a tail end of the endoscope, and adjusting a photographing angle of the endoscope. The present invention has a simple structure, and achieves a function of adjusting a bending angle of the endoscope without increasing a mass and a size of a body of the endoscope.

As a further improvement of the foregoing solution, the deformation bending unit includes two semicircular electro-deformation pieces made of the electro-deformation material, side edges of the electro-deformation pieces are provided with the insulating layer, and upper and lower ends of the electro-deformation pieces are both provided with the electrodes. By controlling the voltage of the electrodes, one of the two electro-deformation pieces is contracted and the other one is stretched, so that the deformation bending unit bends and turns in two different directions respectively.

As a further improvement of the foregoing solution, the deformation bending unit includes four quarter-round electro-deformation pieces made of the electro-deformation material, side edges of the electro-deformation pieces are provided with the insulating layer, and upper and lower ends of the electro-deformation pieces are both provided with the electrodes. By controlling the voltage of the electrodes, a degree of contracting and stretching of the electro-deformation pieces is controlled, so that the deformation bending unit bends and turns in four different directions respectively.

As a further improvement of the foregoing solution, the electrodes are graphite electrodes. The graphite electrode is easy to process and light in weight.

As a further improvement of the foregoing solution, the insulating layer is made of a PET material. The PET has good rub resistance, low abrasion and high hardness, and has the greatest toughness among thermoplastics. The PET has good electrical insulation property, may not crack when the electro-deformation pieces are deformed, and has a long service life.

As a further improvement of the foregoing solution, the electroactive polymer is one of an electrostrictive graft elastomer, an electrostrictive film or a ferroelectric polymer. When deformed under the action of an electric field, these polymer materials have greater strain capacity, light weight, high driving efficiency and good seismic performance.

As a further improvement of the foregoing solution, the voltage generating unit is provided on the handle portion.

### BRIEF DESCRIPTION OF THE DRAWINGS

The present invention is further described hereinafter with reference to the drawings and the embodiments.
FIG. 1 is a schematic structural diagram of an endoscope according to a preferred embodiment of the present invention;
FIG. 2 is a schematic structural diagram of an electro-deformation bending tube according to a preferred embodiment of the present invention;
FIG. 3 is a schematic structural diagram of a deformation bending unit according to a preferred embodiment of the present invention;
FIG. 4 is an axial sectional view of the deformation bending unit according to an preferred embodiment of the present invention;
FIG. 5 is a schematic structural diagram of a deformation bending unit according to another preferred embodiment of the present invention; and
FIG. 6 is an axial sectional view of a deformation bending unit according to another preferred embodiment of the present invention.

### DETAILED DESCRIPTION

The detailed embodiments of the present invention will be described in detail, the preferred embodiments of the present invention are shown in the drawings, the drawings are intended to supplement the description in the text part of the specification with figures, so that people can intuitively and vividly understand each technical feature and the overall technical solution of the present invention, but it shall not be understood as a limitation to the protection scope of the present invention.

In the description of the present invention, it shall be understood that the orientation or position relation related to the orientation description, such as the orientation or position relation indicated by the terms upper, lower, front, rear, left, right, etc., is based on the orientation or position relation shown in the drawings, which is only used for convenience of description of the present invention and simplification of description instead of indicating or implying that the indicated device or element must have a specific orientation, and be constructed and operated in a specific orientation, and thus shall not be understood as a limitation to the present invention.

In the description of the present invention, the meaning of several refers to be one or more, and the meaning of multiple refers to be two or more. The meanings of greater than, less than, more than, etc., are understood as not including the number that follows, while the meanings of above, below, within, etc., are understood as including the number that follows. If there is a description to the first and second, it is only for the purpose of distinguishing between technical features, and shall not be understood as indicating or implying relative importance, implicitly indicating the number of the indicated technical features or implicitly indicating the order of the indicated technical features.

In the description of the present invention, unless otherwise explicitly defined, words such as setting, installing and connecting should be understood in a broad sense, and those having ordinary skills in the art can reasonably determine the specific meanings of the above words in the present invention in combination with the specific contents of the technical solutions.

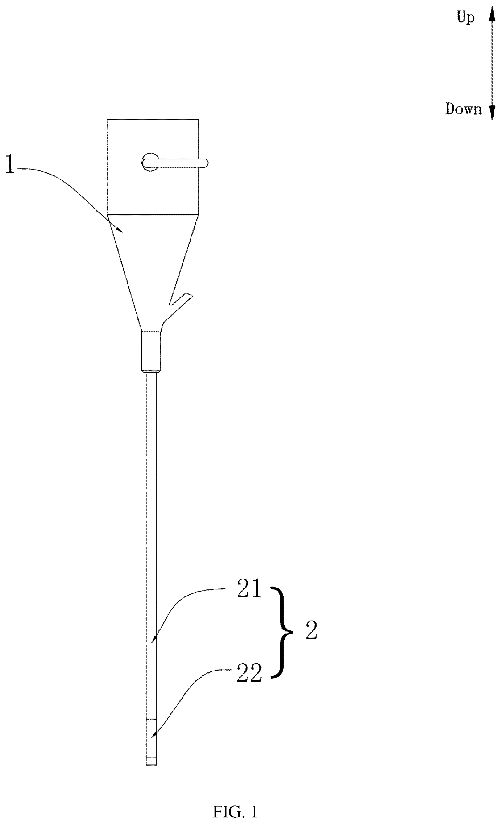

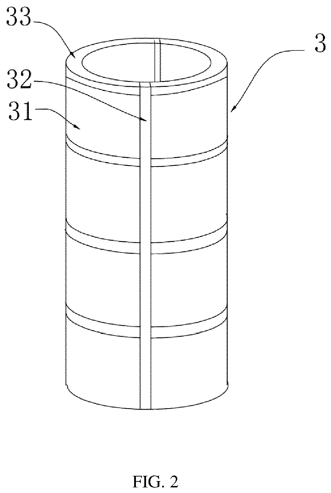

Referring to FIG. 1 and FIG. 2, an electro-deformation bending endoscope includes a handle portion 1 and an insertion portion 2 provided below the handle portion 1. The insertion portion2 includes a straight tube 21 and an electro-deformation bending tube 22, a lower end of the straight tube is coaxially fixed with an upper end of the electro-deformation bending tube 22, and the electro-deformation bending tube 22 includes a plurality of deformation bending units 3 connected end to end. In this embodiment, the number of the deformation bending unit is 4, and in other embodiments, the number of the deformation bending unit 3 may also be an integer greater than 0. Each deformation bending unit 3 includes a plurality of electro-deformation pieces 31 made of an electro-deformation material, an insulating layer 32 is provided between the electro-deformation pieces 31, and a material of the insulating layer 32 needs to have certain stretchability. Electrodes 33 are provided at two ends of each electro-deformation piece 31, and the electrodes 33 are connected with a voltage generating unit (not shown in the figures) through a wire. The voltage generating unit (not shown in the figures) may be provided on the handle portion 1 or other positions of the endoscope or provided independently. The electroactive polymer is one of an electrostrictive graft elastomer, an electrostrictive film or a ferroelectric polymer. When deformed under the action of an electric field, these polymer materials have great strain capacity, light weight, high driving efficiency and good anti-vibration performance. The voltage generating unit (not shown in the figures) is used to output a regulated voltage to the electrodes 33, and the voltage output by the voltage generating unit is controlled by a single chip microcomputer, which controls the voltage applied to the electrodes 33. According to the present invention, the voltage is applied to the electrodes 33 to control the bending of the electro-deformation pieces 31, so as to bend a tail end of the endoscope and adjust a photographing angle of the endoscope. The present invention has a simple structure, and achieves a function of adjusting a bending angle of the endoscope without increasing a mass and a size of a body of the endoscope.

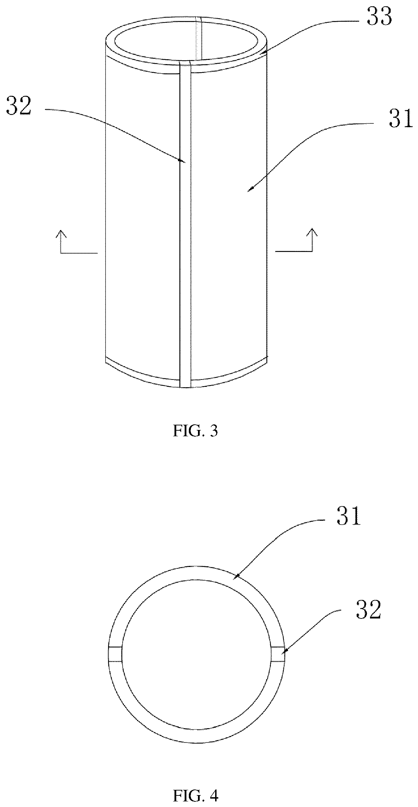

Referring to FIG. 3 and FIG. 4, in some embodiments, the deformation bending unit 3 includes two semicircular electro-deformation pieces 31 made of the electro-deformation material, the two electro-deformation pieces 31 enclose to define a cylindrical shape, and side edges of the electro-deformation pieces 31 are provided with an insulating layer 32 made of a PET material. The PET has good rub resistance, low abrasion and high hardness, and has the greatest toughness among thermoplastics. The **PET** has good electrical insulation property, may not crack when the electro-deformation pieces 31 are deformed, and has a long service life. Upper and lower ends of the electro-deformation pieces 31 are both provided with the electrodes 33, and the electrodes 33 are graphite electrodes 33. The graphite electrode 33 is easy to process and light in weight. By controlling the voltage of the electrodes 33, one of the two electro-deformation pieces 31 is contracted and the other one is stretched, so that the deformation bending unit 3 bends and turns in two different directions respectively. Therefore, the endoscope is adjusted to capture images in different directions.

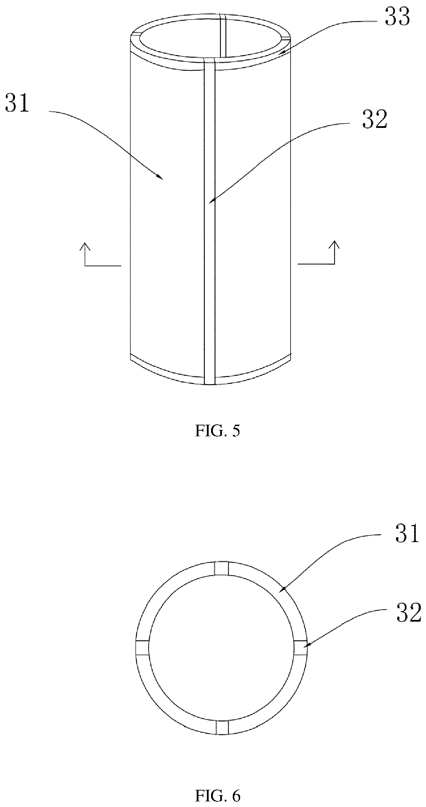

Referring to FIG. 5 and FIG. 6, in some other embodiments, the deformation bending unit 3 includes four quarter-round electro-deformation pieces 31 made of the electro-deformation material, side edges of the electro-deformation pieces 31 are provided with the insulating layer 32, and the insulating layer 32 is made of a **PET** material. The **PET** has good rub resistance, low abrasion and high hardness, and has the greatest toughness among thermoplastics. The **PET** has good electrical insulation property, may not crack when the electro-deformation pieces 31 are deformed, and has a long service life. Upper and lower ends of the electro-deformation pieces 31 are both provided with the electrodes 33, and the electrodes 33 are graphite electrodes 33. By controlling the voltage of the electrodes 33, a degree of contracting and stretching of the electro-deformation pieces 31 is controlled, so that the deformation bending unit 3 bends and turns in four different directions respectively.

The embodiments of the present invention are described in detail with reference to the drawings above, but the present invention is not limited to the above embodiments, and various changes may also be made within the knowledge scope of those of ordinary skills in the art without departing from the purpose of the present invention.

## Claims

1. An electro-deformation bending endoscope, comprising a handle portion (1) and an insertion portion (2) provided below the handle portion (1), wherein the insertion portion (2) comprises a straight tube (21) and an electro-deformation bending tube (22), a lower end of the straight tube (21) is coaxially fixed with an upper end of the electro-deformation bending tube (22), the electro-deformation bending tube (22) comprises a plurality of deformation bending units (3) connected end to end, each deformation bending unit (3) comprises at least two electro-deformation pieces (31) made of an electro-deformation material, an insulating layer (32) is provided between the electro-deformation pieces (31), electrodes (33) are provided at two ends of each electro-deformation piece (31), and the electrodes (33) are connected with a voltage generating unit through a wire.

2. The electro-deformation bending endoscope according to claim 1, wherein: the deformation bending unit (3) comprises two semicircular electro-deformation pieces (31) made of the electro-deformation material, side edges of the electro-deformation pieces (31) are provided with the insulating layer (32), and upper and lower ends of the electro-deformation pieces (31) are both provided with the electrodes (33).

3. The electro-deformation bending endoscope according to claim 1, wherein: the deformation bending unit (3) comprises four quarter-round electro-deformation pieces (31) made of the electro-deformation material, side edges of the electro-deformation pieces (31) are provided with the insulating layer (32), and upper and lower ends of the electro-deformation pieces (31) are both provided with the electrodes (33).

4. The electro-deformation bending endoscope according to claim 1, wherein: the electrodes (33) are graphite electrodes (33).

5. The electro-deformation bending endoscope according to claim 1, wherein: the insulating layer (32) is made of a PET material.

6. The electro-deformation bending endoscope according to claim 1, wherein: the electro-deformation material is an electroactive polymer which is one of an electrostrictive graft elastomer, an electrostrictive film or a ferroelectric polymer.

7. The electro-deformation bending endoscope according to claim 1, wherein: the voltage generating unit is provided on the handle portion (1).

## Patentansprüche

1. Biegsames Elektrodeformationsendoskop, umfassend einen Griffabschnitt (1) und einen unterhalb des Griffabschnitts (1) bereitgestellten Einführabschnitt (2), wobei der Einführabschnitt (2) ein gerades Rohr (21) und ein biegsames Elektrodeformationsrohr (22) umfasst, ein unteres Ende des geraden Rohrs (21) koaxial an einem oberen Ende des biegsamen Elektrodeformationsrohrs (22) befestigt ist, das biegsame Elektrodeformationsrohr (22) eine Vielzahl von Ende zu Ende verbundener biegsamer Deformationseinheiten (3) umfasst, jede biegsame Deformationseinheit (3) mindestens zwei aus einem Elektrodeformationsmaterial hergestellte Elektrodeformationsteile (31) umfasst, eine Isolierschicht (32) zwischen den Elektrodeformationsteilen (31) bereitgestellt ist, Elektroden (33) an zwei Enden jedes Elektrodeformationsteils (31) bereitgestellt sind und die Elektroden (33) über einen Draht mit einer Spannungserzeugungseinheit verbunden sind.

2. Biegsames Elektrodeformationsendoskop nach Anspruch 1, wobei: die biegsame Deformationseinheit (3) zwei aus dem Elektrodeformationsmaterial hergestellte halbkreisförmige Elektrodeformationsteile (31) umfasst, Seitenkanten der Elektrodeformationsteile (31) mit der Isolierschicht (32) versehen sind und obere und untere Enden der Elektrodeformationsteile (31) beide mit den Elektroden (33) versehen sind.

3. Biegsames Elektrodeformationsendoskop nach Anspruch 1, wobei: die biegsame Deformationseinheit (3) vier aus dem Elektrodeformationsmaterial hergestellte Viertelkreis-Elektrodeformationsteile (31) umfasst, Seitenkanten der Elektrodeformationsteile (31) mit der Isolierschicht (32) versehen sind und obere und untere Enden der Elektrodeformationsteile (31) beide mit den Elektroden (33) versehen sind.

4. Biegsames Elektrodeformationsendoskop nach Anspruch 1, wobei: die Elektroden (33) Graphitelektroden (33) sind.

5. Biegsames Elektrodeformationsendoskop nach Anspruch 1, wobei: die Isolierschicht (32) aus einem PET-Material hergestellt ist.

6. Biegsames Elektrodeformationsendoskop nach Anspruch 1, wobei: das Elektrodeformationsmaterial ein elektroaktives Polymer ist, das eines von einem elektrostriktiven Pfropfelastomer, einem elektrostriktiven Film oder einem ferroelektrischen Polymer ist.

7. Biegsames Elektrodeformationsendoskop nach Anspruch 1, wobei: die Spannungserzeugungseinheit am Griffabschnitt (1) bereitgestellt ist.

## Revendications

1. Endoscope à flexion par électro-déformation, comprenant une partie poignée (1) et une partie d'insertion (2) prévue sous la partie poignée (1), dans lequel la partie d'insertion (2) comprend un tube droit (21) et un tube de flexion par électro-déformation (22), une extrémité inférieure du tube droit (21) est fixée coaxialement avec une extrémité supérieure du tube de flexion par électro-déformation (22), le tube de flexion par électro-déformation (22) comprend une pluralité d'unités de flexion par déformation (3) reliées bout à bout, chaque unité de flexion par déformation (3) comprend au moins deux pièces d'électro-déformation (31) constituées d'un matériau d'électro-déformation, une couche isolante (32) est prévue entre les pièces d'électro-déformation (31), des électrodes (33) sont prévues au niveau de deux extrémités de chaque pièce d'électro-déformation (31), et les électrodes (33) sont reliées à une unité de génération de tension par un fil.

2. Endoscope à flexion par électro-déformation selon la revendication 1, dans lequel : l'unité de flexion par déformation (3) comprend deux pièces d'électro-déformation (31) semi-circulaires constituées du matériau d'électro-déformation, des bords latéraux des pièces d'électro-déformation (31) sont prévus avec la couche isolante (32), et des extrémités supérieure et inférieure des pièces d'électro-déformation (31) sont toutes deux prévues avec les électrodes (33).

3. Endoscope à flexion par électro-déformation selon la revendication 1, dans lequel : l'unité de flexion par déformation (3) comprend quatre pièces d'électro-déformation (31) quart-de-rond constituées du matériau d'électro-déformation, des bords latéraux des pièces d'électro-déformation (31) sont prévus avec la couche isolante (32), et des extrémités supérieure et inférieure des pièces d'électro-déformation (31) sont toutes deux prévues avec les électrodes (33).

4. Endoscope à flexion par électro-déformation selon la revendication 1, dans lequel : les électrodes (33) sont des électrodes en graphite (33).

5. Endoscope à flexion par électro-déformation selon la revendication 1, dans lequel : la couche isolante (32) est constituée d'un matériau PET.

6. Endoscope à flexion par électro-déformation selon la revendication 1, dans lequel : le matériau d'électro-déformation est un polymère électroactif qui est l'un d'un élastomère greffé électrostrictif, d'un film électrostrictif ou d'un polymère ferroélectrique.

7. Endoscope à flexion par électro-déformation selon la revendication 1, dans lequel : l'unité de génération de tension est prévue sur la partie poignée (1).
